# EUROPEAN PATENT SPECIFICATION

(11) **EP 3 021 894 B1**
(45) Date of publication and mention of the grant of the patent: **05.10.2022**
(21) Application number: 14831565.8
(22) Date of filing: 15.07.2014
(51) Int. Cl.: A61M 25/10, A61F 2/958, A61F 2/95

(54) **BALLOON CATHETER HAVING IMPROVED RETRACTABLE SHEATH AND LOCKING MECHANISM**
BALLONKATHETER MIT VERBESSERTER EINZIEHBARER SCHLEUSE UND VERRIEGELUNGSMECHANISMUS
CATHÉTER À BALLON COMPORTANT UNE GAINE RÉTRACTABLE ET UN MÉCANISME DE VERROUILLAGE PERFECTIONNÉS

(30) Priority: 15.07.2013 US 201361846095 P; 15.06.2014 US 201462012382 P
(43) Date of publication of application: 25.05.2016
(73) Proprietor: Pigott, John, P., Sylvania, OH 43560 (US)
(72) Inventor: Pigott, John, P., Sylvania, OH 43560 (US)
(74) Representative: Haseltine Lake Kempner LLP
(86) International application number: PCT/US2014/046616
(87) International publication number: WO 2015/017118

(56) References cited:
- US-A- 6 071 287
- US-A1- 2004 151 304
- US-A1- 2004 267 345
- US-A1- 2008 140 051
- US-A1- 2009 105 686
- US-A1- 2012 143 054

## Description

### BACKGROUND OF THE INVENTION

This invention relates in general to balloon catheters that are used in **surgical** procedures. In particular, this invention relates to an improved structure for such a **balloon** catheter having a retractable sheath that can quickly and easily adjust the length of the inflated portion of the balloon and a locking mechanism for selectively retaining the sheath in the desired position for use.

In many surgical procedures, such as percutaneous transluminal **angioplasty** procedures, a catheter having a selectively inflatable portion provided thereon (commonly referred to as a balloon catheter) is used to open a blockage and/or place a stent in a blood vessel. To accomplish this, the balloon of the catheter is initially positioned at a desired location within the blood vessel. Then, the balloon of the **catheter** is inflated so as to expand into engagement with an inner surface of the blood vessel, thereby expanding the blockage. If desired, an expandable stent can be disposed aboutthe balloon of the catheter such that when the balloon is inflated, the stent is expanded into engagement with the inner surface of the blood vessel. In either event, the balloon issubsequently deflated after use, allowing the catheter to be removed from the blood vessel.

Although a wide variety of balloon catheters are known in the art, including US Published Application No. 2008/140051 to Bei et. al. published June 12, 2008, US Patent No. 6,071,287 to Verbeek dated June 6, 2000, US Published Application No. 2012/143054 to Eaton et. al. published June 7, 2012, US Published Application No. 2004/267345 to Lorenzo et. al. published December 30, 2004, US Published Application No. 2004/151304 to Boelens et. al. published August 5, 2004 and US Published Application No. 2009/105686 to David Snow published 23 April 2009, it would desirable to provide an improved structure for such a balloon catheter having a retractable sheath that can quickly and easily adjust the length of the inflated portion of the balloon and a locking mechanism for selectively retaining the sheath in the desired position for use.

### SUMMARY OF THE INVENTION

This invention relates to an improved structure for a balloon catheter as set out in claim 1 below. Preferred features of the invention are set out in the dependent claims below.

Various aspects of this invention will become apparent to those skilled in the art from the following detailed description of the preferred embodiments, when read in light of the accompanying drawings.

### BRIEF DESCRIPTION OF THE DRAWINGS

Fig. 1 is a side elevational view of a first end of a balloon catheter assembly in accordance with this **invention.**
Fig. 2 is an enlarged sectional elevational view of a second end of a first embodiment of the balloon catheter assembly illustrated in Fig. 1.

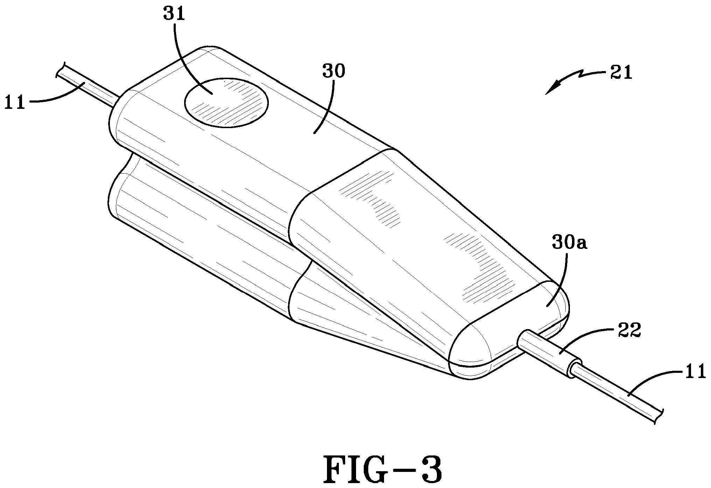

Fig. 3 is a perspective view of a clamp of the balloon catheter **assembly** illustrated in Fig. 1.
Fig. 4 is an enlarged sectional elevational view showing the clamp of the balloon catheter assembly in a locked condition.
Fig. 5 is an enlarged sectional elevational view similar to Fig. 4 showing the clamp of the balloon catheter assembly in an unlocked **condition.**
Fig. 6 is a further enlarged sectional elevational view of a second end of a second embodiment of a balloon catheter assembly in accordance with this invention.
Fig. 7 is an enlarged sectional elevational view similar to Fig. 4 showing a third embodiment of this invention, wherein a series of spaced indentations/protrusions are provided on one side of the movable sheath.
Fig. 8 is an enlarged sectional elevational view similar to Fig. 7 showing a fourth embodiment of this invention, wherein a series of annular spaced indentations/protrusions are provided about the circumference of the movable sheath.
Fig. 9 is a sectional elevational view similar to Fig. 2 showing the end of the sheath in sealing engagement with a tapered insertion tip.
Fig. 10 is an enlarged view of the tapered insertion tip illustrated in Fig. 9.
Fig. 11 is an enlarged view similar to Fig. 10 showing the sheath partially retracted to expose the balloon.

### DETAILED DESCRIPTION OF THE PREFERRED EMBODIMENTS

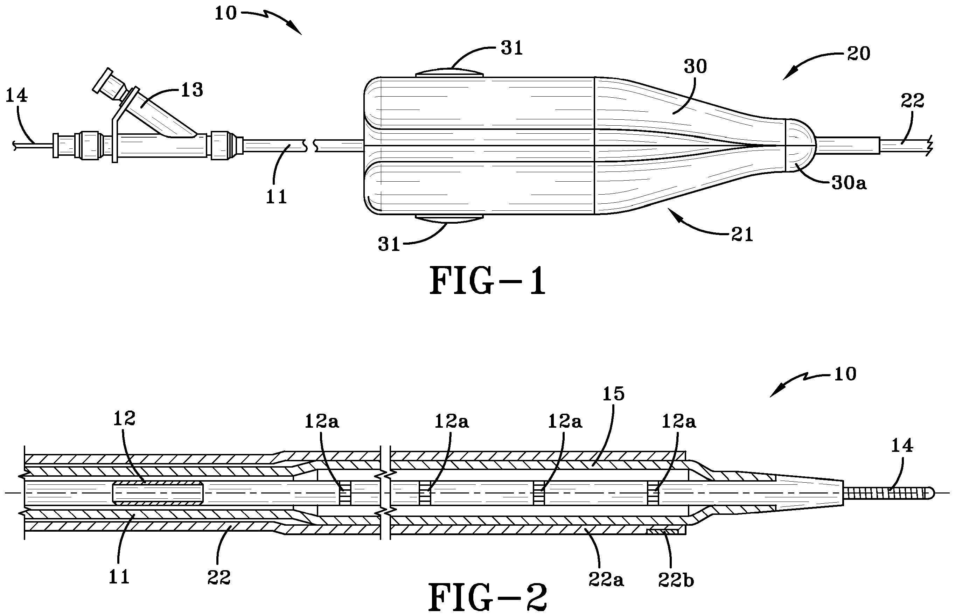

Referring now to the drawings, there is illustrated in Figs. 1 and 2 balloon catheter assembly, indicated generally at 10, in accordance with this invention. The balloon catheter assembly 10 has an elongated shaft portion that includes an outer member 11 and an inner member 12. As best shown in Fig. 2, the outer member 11 and the inner member 12 are both generally tubular in shape, although such is not required, and the inner member 12 is disposed within the outer member 11. Thus, an annular outer lumen is defined between the inner surface of the outer tubular member 11 and the outer surface of the inner tubular member 12. Similarly, a cylindrical inner lumen is defined within the inner tubular member 12. The purpose for the outer and inner lumens will be explained below.

If desired, one or more optional radiopaque markers 12a may be provided on the inner tubular member 12. The radiopaque markers 12a are conventional in the art and are provided to facilitate the determination of the location of the inner tubular member 12 during a procedure using conventional fluoroscopy techniques.

As shown in Fig. 1, an adapter 13 is connected to a first end of the elongated shaft portion of the balloon catheter assembly 10. The adapter 13 is conventional in the art and is provided to facilitate access to both the outer and inner lumens during use of the balloon catheter assembly 10. For example, a guide wire 14 can be inserted into the adapter 13 and through the inner lumen to a second end of the elongated shaft portion of the balloon catheter assembly 10, as shown in Fig. 2. The guide wire 14 is conventional in the art and is provided to facilitate the positioning of the balloon catheter assembly 10 at a desired location for use, such as within a blood vessel as described above.

The outer tubular member 11 includes an inflatable balloon 15. The balloon 15 is conventional in the art and is typically connected to the outer tubular member 11 such that at least a portion thereof extends about an end portion of the inner tubular member 12, as shown in Fig. 2. The interior of the balloon 15 communicates with the outer lumen of the balloon catheter assembly 10. As a result, an inflation fluid can be selectively introduced into the adapter 13 and through the outer lumen to the interior of the balloon 15, causing it to inflate in a known manner. Additionally, the interior of the balloon 15 can be vented through the outer lumen and the adapter 13 to the atmosphere, allowing it to deflate after use.

As shown in Figs. 1 and 2, the balloon catheter assembly 10 includes an adjustable sheath assembly, indicated generally at 20. As will be explained in detail below, the adjustable sheath assembly 20 is provided to selectively define a portion of the balloon 15 that is desired to be inflated during use. To accomplish this, the adjustable sheath assembly 20 includes a clamp, indicated generally at 21, and a sheath 22. As shown in Fig. 1, the clamp 21 is disposed about the outer member 11 adjacent to the adapter 13 and the first end of the elongated shaft portion of the balloon catheter assembly 10. The structure and operation of the clamp 21 will be explained in detail below. The sheath 22 extends from the clamp 21 about the outer member 11 toward the second end of the elongated shaft portion of the balloon catheter assembly 10, as shown in Fig. 2. In the illustrated embodiment, the sheath 22 includes an optional expanded portion 22a that is located adjacent to the second end of the elongated shaft portion of the balloon catheter assembly 10, as shown in Fig. 2. One or more optional radiopaque markers 22b may be provided on the expanded portion 22a of the sheath 22 to facilitate the determination of the location of the inner tubular member 12 using conventional fluoroscopy techniques during use. Also, the expanded portion 22a of the sheath 22 may have an elastically expandable distal tip (not shown) provided thereon that is expanded in a conventional manner when the balloon 15 is inflated.

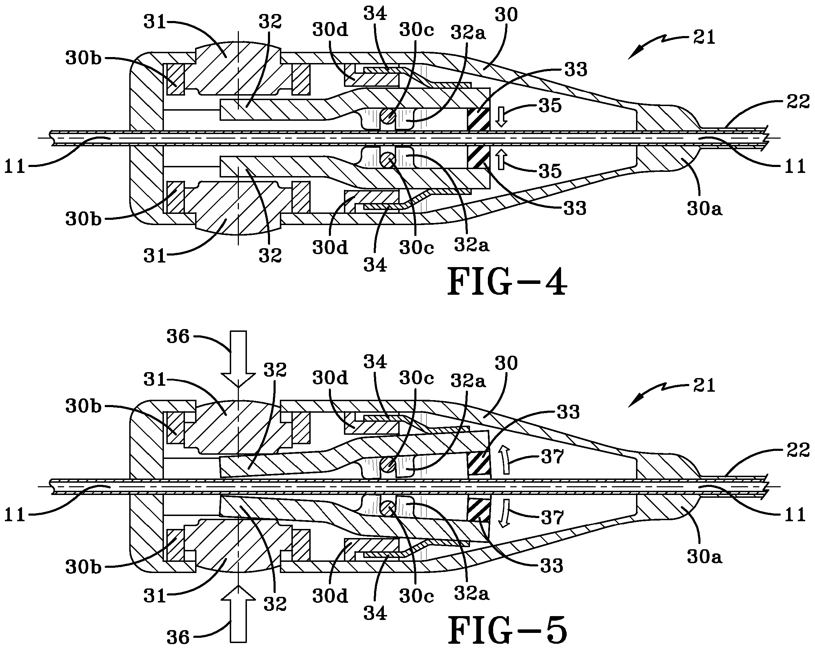

Figs. 4 and 5 illustrate the internal structure of the clamp 21 of the adjustable sheath assembly 20. As shown therein, the clamp 21 includes a housing 30 through which the outer member 11 of the elongated shaft portion of the balloon catheter assembly 10 extends. The housing 30 includes a tip 30a that is connected to the sheath 22 of the adjustable sheath assembly. Thus, the housing 30 of the clamp 21 is connected to the sheath 22 for movement therewith, as will be explained in detail below.

The housing 30 of the clamp 21 also includes a pair of button supports 30b, within which respective manually operable push buttons 31 are supported for inward and outward movement relative to the housing 30. Each of the button supports 30b is generally hollow and cylindrical in shape, and each of the push buttons 31 is generally cylindrical in shape. However, the button supports 30b and the push buttons 31 may have any desired shape or combination of shapes. The purpose for the push buttons 31 will be explained below.

The housing 30 of the clamp 21 further includes a pair of actuator arm supports 30c, upon which respective actuator arms 32 are supported for pivoting movement relative to the housing 30. Each of the actuator arm supports 30c is a generally cylindrical protrusion that extends inwardly within the housing 30. However, the actuator arm supports 30c may have any desired shape or combination of shapes. Each of the illustrated actuator arms 32 is provided with a pair of legs 32a that define a pocket. The actuator arm supports 30c of the actuator arms 32 are respectively received within the pockets defined by the pairs of legs 32a of the actuator arms 32. Thus, as will be explained in detail below, the actuator arms 32 are respectively supported on the actuator arm supports 30c of the housing 30 for independent pivoting movement relative thereto.

As shown in Figs. 4 and 5, first ends of the actuator arms 32 are respectively disposed adjacent to the inner ends of the push buttons 31. The actuator arms 32 also include respective second ends that are disposed remotely from the first ends. Respective engagement pads 33 are secured to the inner surfaces of the second ends of the actuator arms 32. The engagement pads 33 may be formed from any desired material or combination of materials and may, if desired, be omitted entirely. However, it is preferred that the engagements pads 33 be formed from a material having a relatively high coefficient of friction, such as rubber. The purpose for the engagement pads 33 will be explained below.

Lastly, the housing 30 of the clamp 21 includes a pair of springs 34 that independently bias the two actuator arms 32 toward each other, as shown in Fig. 4. In the illustrated embodiment, each of the springs 34 is formed from a flat piece of resilient material, such as a metallic material, that has been deformed to achieve a desired shape. However, the springs 34 may be formed from any desired material or combination of materials. The springs 34 have first ends that are received within respective spring supports 30d provided on the inner surface of the housing 30. The springs 34 also have second ends that bear inwardly upon respective outer surfaces of the second ends of the actuator arms 32. The purpose for the springs 34 will be explained below.

Fig. 4 illustrates the clamp 21 of the balloon catheter assembly 10 in a locked condition. In this locked condition, the second ends of the springs 34 bear inwardly upon respective outer surfaces of the second ends of the actuator arms 32. As a result, the engagements pads 33 are urged inwardly toward one another, as shown at 35, so as to engage opposed portions of the outer surface of the outer member 11 of the balloon catheter assembly 10, which extends through the interior of the housing 30 of the clamp 21. As mentioned above, the engagements pads 33 are preferably formed from a material having a relatively high coefficient of friction. Thus, when the clamp 21 of the balloon catheter assembly 10 in the locked condition, the outer member 11 of the balloon catheter assembly 10 is prevented from moving axially relative to the housing 30 of the clamp 21. As mentioned above, however, the housing 30 of the clamp 21 is connected at the tip 30a to the sheath 22 for movement therewith. Accordingly, when the clamp 21 of the balloon catheter assembly 10 in the locked condition, the outer member 11 of the balloon catheter assembly 10 is also prevented from moving axially relative to the sheath 22.

Fig. 5 illustrates the clamp 21 of the balloon catheter assembly 10 in an unlocked condition. To achieve this unlocked condition, a user of the balloon catheter assembly 10 applies inwardly directed forces, as shown at 36, against the manually operable push buttons 31. These forces overcome the forces exerted by the springs 34 and cause the actuator arms 32 to pivot such that the engagements pads 33 are moved out of engagement, as shown at 37, with the opposed portions of the outer surface of the outer member 11 of the balloon catheter assembly 10. Thus, when the clamp 21 of the balloon catheter assembly 10 in the unlocked condition, the outer member 11 of the balloon catheter assembly 10 is permitted to move axially relative to the housing 30 of the clamp 21. As mentioned above, however, the housing 30 of the clamp 21 is connected at the tip 30a to the sheath 22 for movement therewith. Accordingly, when the clamp 21 of the balloon catheter assembly 10 in the unlocked condition, the outer member 11 of the balloon catheter assembly 10 is also permitted to move axially relative to the sheath 22.

Although it has been described in the context of the illustrated clamp 21, it will be appreciated that it may be practiced with any other desired structure for preventing the balloon 15 from slipping in relation to the sheath 22. For example, a variety of known structures may be used for this purpose. Some of such known structures include a duckbill pinch, a spring-activated clamp, a screw mechanism onto catheter, a Tuohy-Borst type of valve, and the like. Furthermore, it is possible to practice this invention without any clamp 21.

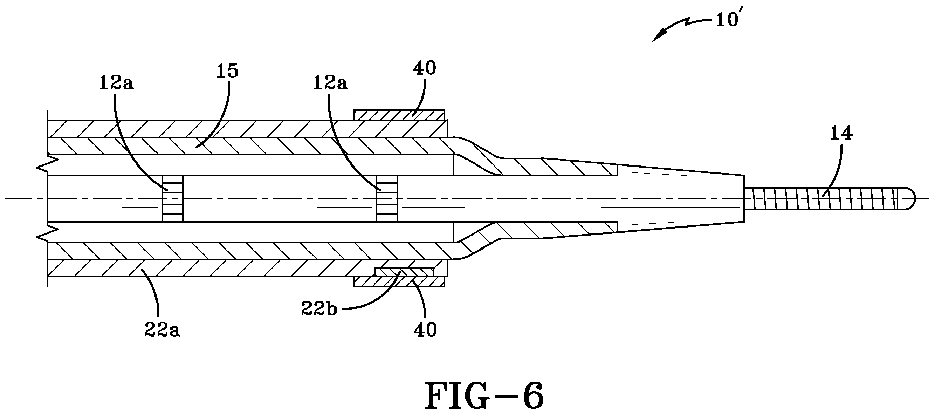

Fig. 6 illustrates a second end of a balloon catheter assembly, indicated generally at 10'. Like reference numbers are used in Fig. 6 to indicate components that are the same as described above. As shown therein, the second end of the modified balloon catheter assembly 10' has a collar 40 provided thereon. The purpose of the collar 40 is to prevent the sheath 22 from splitting or otherwise being deformed or damaged as a result of the high pressure force exerted by the balloon 15 when it is inflated. The illustrated collar 40 is tubular in shape and extends completely about the end of the sheath 22 where the balloon 15 is located. However, it will be appreciated that the collar 40 may have any desired shape and may be located at any desired position on the sheath 22. The collar 40 may be formed from any desired material, such as a metallic material, a high strength aramid fiber material (such as is commercially available under the Kevlar^{®} brand name), or a high durometer plastic material. If desired, the collar 40 may be formed from a radiopaque material, allowing it to function as a marker for the distal tip of the sheath 22. If desired, the distal tip of the sheath 22 may be provided with a smooth edge (not shown) so as not to present a sharp edge toward the balloon 15.

Preferably, the tip of the balloon 15 fits snugly into the end of the sheath 22 so as to provide a watertight engagement therebetween. Such a watertight engagement would maintain the interior compartment of the balloon catheter assembly 10 dry during its initial insertion within a blood vessel or other portion of a body. Maintaining the interior compartment of the balloon catheter assembly 10 dry facilitates the use of conventional drug-coated balloons, stents, and other devices. The coatings provided on such devices are, in some instances, activated by contact with blood and water. In known catheter assemblies, a significant amount of the drugs provided on the balloons, stents, and other devices can be eluted during the initial insertion of the catheter assembly through blood vessels having flowing blood. The watertight engagement of this invention allows for minimal elution of the drugs during the initial insertion of the balloon catheter assembly 10 and maximal delivery of the drugs to the desired site.

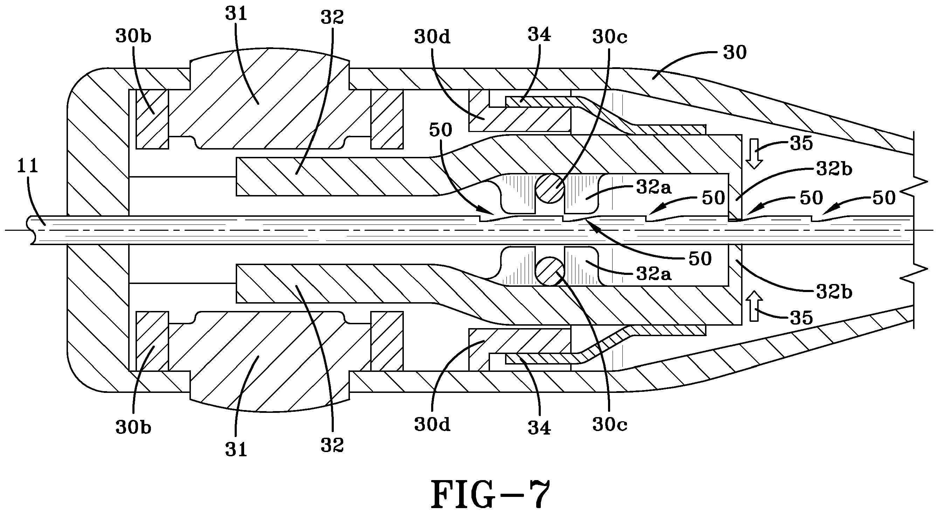

Fig. 7 is an enlarged sectional elevational view similar to Fig. 4 showing the embodiment of this invention, wherein one side of the outer member 11 is provided with a plurality of spaced indentations or protrusions, indicated generally at 50. The indentations or protrusions 50 may have any desired shape or size (or combination of shapes and sizes) and may be provided at any desired location on the one side of the outer member 11. The indentations or protrusions 50 are adapted to cooperate with one or more inwardly extending portions 32b provided on either or both of the actuator arms 32. Thus, when the clamp 21 of the balloon catheter assembly 10 in the locked condition, the inwardly extending portions 32b provided on either or both of the actuator arms 32 engage one or more of the indentations or protrusions 50. As a result, the outer member 11 of the balloon catheter assembly 10 is prevented from moving axially relative to the housing 30 of the clamp 21. Conversely, when the clamp 21 of the balloon catheter assembly 10 in the unlocked condition, the inwardly extending portions 32b provided on either or both of the actuator arms 32 do not engage one or more of the indentations or protrusions 50. As a result, the outer member 11 of the balloon catheter assembly 10 is permitted to move axially relative to the housing 30 of the clamp 21.

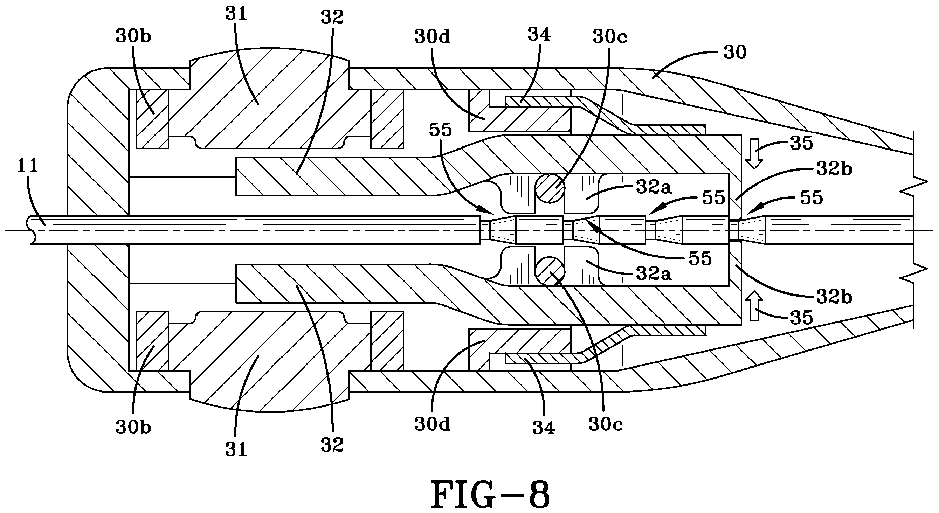

Fig. 8 is an enlarged sectional elevational view similar to Fig. 7 showing a second embodiment of this invention, wherein the outer member 11 is provided with a series of annular spaced indentations or protrusions, indicated generally at 55. The indentations or protrusions 55 may have any desired shape or size (or combination of shapes and sizes) and may be provided at any desired location on the outer member 11. The indentations or protrusions 55 are adapted to cooperate with one or more inwardly extending portions 32b provided on either or both of the actuator arms 32. Thus, when the clamp 21 of the balloon catheter assembly 10 in the locked condition, the inwardly extending portions 32b provided on either or both of the actuator arms 32 engage one or more of the indentations or protrusions 55. As a result, the outer member 11 of the balloon catheter assembly 10 is prevented from moving axially relative to the housing 30 of the clamp 21. Conversely, when the clamp 21 of the balloon catheter assembly 10 in the unlocked condition, the inwardly extending portions 32b provided on either or both of the actuator arms 32 do not engage one or more of the indentations or protrusions 55. Asa result, the outer member 11 of the balloon catheter assembly 10 is permitted to move axially relative to the housing 30 of the clamp 21.

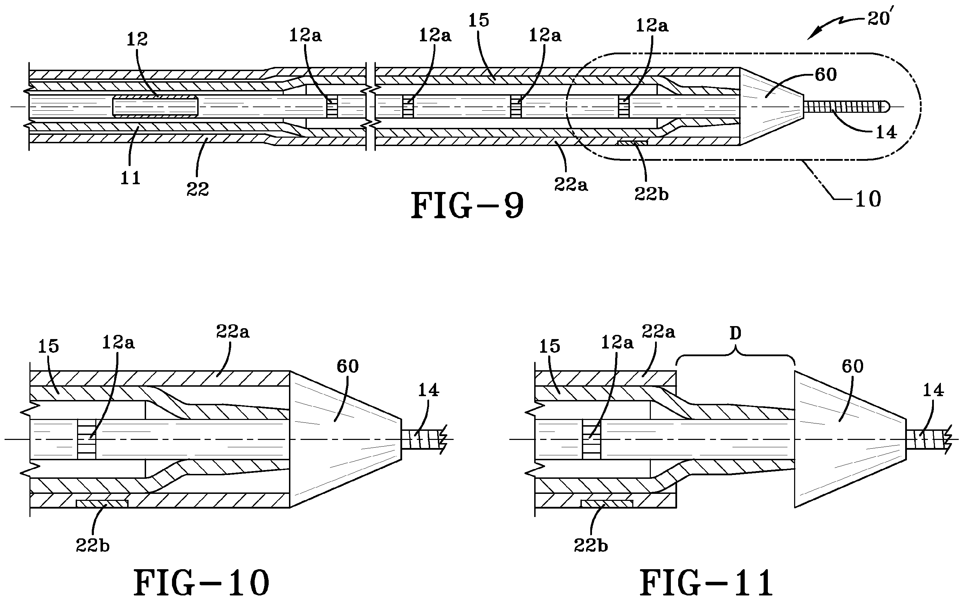

Figs. 9, 10, and 11 show a modified end of the adjustable sheath assembly 20' with a tapered insertion tip 60. In Figs. 9 and 10, the end of the adjustable sheath assembly 20 in sealing engagement with the tapered insertion tip 60. Fig. 11 is similar to Fig. 10, but shows the end of the adjustable sheath assembly 20 partially retracted to expose the balloon 15 in the manner described above.

The principle and mode of operation of this invention have been explained and illustrated in its preferred embodiments. However, it must be understood that this invention may be practiced otherwise than as specifically explained and illustrated without departing from the scope of the claims below.

## Claims

1. A balloon catheter assembly comprising:
an inner member (12);
an outer member (11) disposed about the inner member (12) and including a series of spaced apart indentations or protrusions (50) and a balloon (15) having a proximal end and a distal end, wherein the proximal end of the balloon (15) is connected to the outer member (11) and the distal end of the balloon (15) is connected to the inner member (12);
a sheath (22) disposed about the outer member (11) for movement relative to the balloon (15), wherein the sheath (22) is configured to limit the length of the inflated portion of the balloon (15) by to selectively exposing some of the balloon (15) for inflation; and
a clamp (21) comprising a housing (30) and a first and a second actuator arm (32), wherein said first and a second actuator arm (32) are supported within said housing (30) and are selectively movable into engagement with the indentations or protrusions (50) of the outer member (11) to selectively secure the sheath (22) at a desired position relative to the balloon (15).

2. The balloon catheter assembly defined in Claim 1 wherein
the housing (30) is connected to the sheath (22), and the outer member (11) extends through said housing (30).

3. The balloon catheter assembly defined in Claim 2 wherein
the housing (30) has first and second actuator arms supports (30c) provided therein, and wherein the first and second actuator arms (32) are respectively supported on the first and second actuator arm supports (30c); and
the first and second actuator arms (32) include respective engagement pads (33) that are selectively movable into engagement with the outer member (11).

4. The balloon catheter assembly defined in Claim 2
wherein each of the first and second actuator arms (32) is biased into engagement with the outer member (11); and
further comprising first and second springs (34) that are supported on the housing (30) and respectively bias the first and second actuator arms (32) into engagement with the outer member (11).

5. The balloon catheter assembly defined in Claim 2 further including
first and second push buttons (30b) that are operable to respectively move the first and second actuator arms (32) out of engagement with the outer member (11).

6. The balloon catheter assembly defined in claim 1 wherein
the balloon tip (30a) fits into the end of the sheath (22) in a smooth transition.

7. The balloon catheter assembly defined in claim 1 wherein
the balloon (15) fits snugly into the end of the sheath (22) so as to provide a watertight engagement therebetween.

8. The balloon catheter assembly defined in claim 7 further comprising
a drug coating provided on the outer surface of the balloon (15).

9. The balloon catheter assembly defined in claim 1 further comprising
a collar (40) provided about an end of the balloon catheter assembly and configured to provide additional strength to the sheath (22).

10. The balloon catheter assembly defined in claim 1 further comprising
a tapered insertion tip (60) provided on the distal end of the balloon (15).

11. The balloon catheter assembly defined in claim 1 further comprising
a series of radiopaque marker bands (12a) located at select intervals on the inner member (12).

12. The balloon catheter assembly defined in claim 1 further comprising
a series of radiopaque marker bands (12a) located at select intervals on an expanded portion (22a) of the sheath (22).

## Patentansprüche

1. Ballonkatheteranordnung umfassend: ein inneres Element (12);
ein äußeres Element (11), das um das innere Element (12) angeordnet ist und eine Reihe von beabstandeten Vertiefungen oder Vorsprüngen (50) beinhaltet, und einen Ballon (15), der ein proximales Ende und ein distales Ende aufweist, wobei das proximale Ende des Ballons (15) mit dem äußeren Element (11) verbunden ist und das distale Ende des Ballons (15) mit dem inneren Element (12) verbunden ist;
eine Hülle (22), die für eine Bewegung in Bezug auf den Ballon (15) um das äußere Element (11) angeordnet ist, wobei die Hülle (22) konfiguriert ist, um die Länge des aufgeblasenen Abschnitts des Ballons (15) zu begrenzen, indem sie selektiv etwas von dem Ballon (15) zum Aufblasen freilegt; und
eine Klammer (21), umfassend ein Gehäuse (30) und einen ersten und einen zweiten Betätigungsarm (32), wobei der erste und der zweite Betätigungsarm (32) innerhalb des Gehäuses (30) gehalten werden und selektiv in Eingriff mit den Vertiefungen oder Vorsprüngen (50) des äußeren Elements (11) bewegt werden können, um die Hülle (22) selektiv in einer gewünschten Position in Bezug auf den Ballon (15) zu fixieren.

2. Ballonkatheteranordnung gemäß Anspruch 1, wobei
das Gehäuse (30) mit der Hülle (22) verbunden ist und das äußere Element (11) sich durch das Gehäuse (30) erstreckt.

3. Ballonkatheteranordnung gemäß Anspruch 2, wobei
das Gehäuse (30) einen ersten und einen zweiten Betätigungsarmträger (30c) aufweist, die darin bereitgestellt sind, und wobei der erste und der zweite Betätigungsarm (32) jeweils an dem ersten und dem zweiten Betätigungsarmträger (30c) gehalten werden; und
der erste und der zweite Betätigungsarm (32) jeweils Eingriffspads (33) beinhaltet, die selektiv in Eingriff mit dem äußeren Element (11) bewegt werden können.

4. Ballonkatheteranordnung gemäß Anspruch 2
wobei jeder von dem ersten und dem zweiten Betätigungsarm (32) in Eingriff mit dem äußeren Element (11) vorgespannt ist; und
ferner umfassend eine erste und eine zweite Feder (34), die am Gehäuse (30) abgestützt sind und jeweils den ersten und den zweiten Betätigungsarm (32) in Eingriff mit dem äußeren Element (11) vorspannen.

5. Ballonkatheteranordnung gemäß Anspruch 2, die ferner Folgendes beinhaltet einen ersten und einen zweiten Druckknopf (30b), die betätigt werden können, um jeweils den ersten und den zweiten Betätigungsarm (32) außer Eingriff mit dem äußeren Element (11) zu bringen.

6. Ballonkatheteranordnung gemäß Anspruch 1, wobei
die Ballonspitze (30a) in einem fließenden Übergang in das Ende der Hülle (22) passt.

7. Ballonkatheteranordnung gemäß Anspruch 1, wobei
der Ballon (15) eng sitzend in das Ende der Hülle (22) passt, um eine wasserdichte Verbindung dazwischen bereitzustellen.

8. Ballonkatheteranordnung gemäß Anspruch 1, ferner umfassend eine Arzneimittelbeschichtung, die auf der Außenfläche des Ballons (15) bereitgestellt ist.

9. Ballonkatheteranordnung gemäß Anspruch 1, ferner umfassend einen Bund (40), der um ein Ende der Ballonkatheteranordnung bereitgestellt und konfiguriert ist, um der Hülle (22) zusätzliche Festigkeit zu verleihen.

10. Ballonkatheteranordnung gemäß Anspruch 1, ferner umfassend eine sich verjüngende Einführspitze (60), die an dem distalen Ende des Ballons (15) bereitgestellt ist.

11. Ballonkatheteranordnung gemäß Anspruch 1, ferner umfassend eine Reihe von röntgendichten Markierungsstreifen (12a), die sich in bestimmten Abständen an dem inneren Element (12) befinden.

12. Ballonkatheteranordnung gemäß Anspruch 1, ferner umfassend eine Reihe von röntgendichten Markierungsstreifen (12a), die sich in ausgewählten Abständen an einem erweiterten Abschnitt (22a) der Hülle (22) befinden.

## Revendications

1. Un ensemble cathéter à ballonnet comprenant :
un élément intérieur (12) ;
un élément extérieur (11) disposé autour de l'élément intérieur (12) et comprenant une série d'indentations ou de saillies espacées (50) et un ballonnet (15) ayant une extrémité proximale et une extrémité distale, dans lequel l'extrémité proximale du ballonnet (15) est reliée à l'élément extérieur (11) et l'extrémité distale du ballonnet (15) est reliée à l'élément intérieur (12) ;
une gaine (22) disposée autour de l'élément extérieur (11) pour un mouvement par rapport au ballonnet (15), dans laquelle la gaine (22) est configurée pour limiter la longueur de la partie gonflée du ballonnet (15) en exposant sélectivement une partie du ballonnet (15) pour le gonflage ; et
une pince (21) comprenant un logement (30) et un premier et un deuxième bras d'actionnement (32), dans laquelle lesdits premier et deuxième bras d'actionnement (32) sont supportés à l'intérieur dudit logement (30) et sont sélectivement mobiles pour venir en prise avec les indentations ou les saillies (50) de l'élément extérieur (11) pour fixer sélectivement la gaine (22) à une position souhaitée par rapport au ballonnet (15).

2. L'ensemble cathéter à ballonnet défini dans la revendication 1, dans lequel le logement (30) est relié à la gaine (22), et l'élément extérieur (11) s'étend à travers ledit logement (30).

3. L'ensemble cathéter à ballonnet défini dans la revendication 2, dans lequel
le logement (30) présente des premier et deuxième supports de bras d'actionnement (30c) prévus à l'intérieur, et dans lequel les premier et deuxième bras d'actionnement (32) sont respectivement supportés sur les premier et deuxième supports de bras d'actionnement (30c) ; et
les premier et deuxième bras d'actionnement (32) comprennent des patins d'engagement respectifs (33) qui sont sélectivement mobiles pour venir en prise avec l'élément extérieur (11).

4. L'ensemble cathéter à ballonnet défini dans la revendication 2
dans lequel chacun des premier et deuxième bras d'actionnement (32) est sollicité en prise avec l'élément extérieur (11) ; et
comprenant en outre des premier et deuxième ressorts (34) qui sont supportés sur le logement (30) et sollicitent respectivement les premier et deuxième bras d'actionnement (32) en prise avec l'élément extérieur (11).

5. L'ensemble cathéter à ballonnet défini dans la revendication 2 comprenant en outre
des premier et deuxième boutons poussoirs (30b) qui peuvent être actionnés pour déplacer respectivement les premier et deuxième bras d'actionnement (32) hors de prise avec l'élément extérieur (11).

6. L'ensemble cathéter à ballonnet défini dans la revendication 1, dans lequel l'extrémité du ballonnet (30a) s'adapte dans l'extrémité de la gaine (22) en une transition douce.

7. L'ensemble cathéter à ballonnet défini dans la revendication 1, dans lequel le ballonnet (15) s'adapte parfaitement à l'extrémité de la gaine (22) de manière à fournir un engagement étanche à l'eau entre eux.

8. L'ensemble cathéter à ballonnet défini dans la revendication 7 comprenant en outre
un enrobage de médicament prévu sur la surface extérieure du ballonnet (15).

9. L'ensemble cathéter à ballonnet défini dans la revendication 1 comprenant en outre
un collier (40) prévu autour d'une extrémité de l'ensemble cathéter à ballonnet et configuré pour fournir une résistance supplémentaire à la gaine (22).

10. L'ensemble cathéter à ballonnet défini dans la revendication 1 comprenant en outre
une pointe d'insertion effilée (60) prévue sur l'extrémité distale du ballonnet (15).

11. L'ensemble cathéter à ballonnet défini dans la revendication 1 comprenant en outre
une série de bandes de marquage radio-opaques (12a) situées à des intervalles choisis sur l'élément intérieur (12).

12. L'ensemble cathéter à ballonnet défini dans la revendication 1 comprenant en outre
une série de bandes de marquage radio-opaques (12a) situées à des intervalles choisis sur une partie élargie (22a) de la gaine (22).
